# EUROPÄISCHE PATENTSCHRIFT

(11) **EP 0 972 055 B1**
(45) Veröffentlichungstag und Bekanntmachung des Hinweises auf die Patenterteilung: **10.05.2006**
(21) Anmeldenummer: 99901597.7
(22) Anmeldetag: 13.01.1999
(51) Int. Cl.: C12N 15/62, C07K 14/72, C07K 19/00, G01N 33/58, G01N 33/53, A61K 38/17

(54) **VERFAHREN ZUR HERSTELLUNG VON GEREINIGTEN UND MARKIERTEN TSH-REZEPTOR-PRÄPARATEN UND DIE VERWENDUNG VON PRÄPARATEN DIESER ART IN der DIAGNOSTIK**
METHOD FOR PRODUCING PURIFIED AND MARKED TSH RECEPTOR PREPARATIONS AND THE USE OF the corresponding PREPARATIONS IN DIAGNOSTICS
PROCEDE DE FABRICATION DE PREPARATIONS PURIFIEES ET MARQUEES DE RECEPTEURS TSH ET UTILISATION DE CES PREPARATIONS EN DIAGNOSTIC

(30) Priorität: 14.01.1998 DE 19801154
(43) Veröffentlichungstag der Anmeldung: 19.01.2000
(73) Patentinhaber: B.R.A.H.M.S Aktiengesellschaft, 16761 Hennigsdorf (DE)
(72) Erfinder: LOOS, Ulrich, D-89081 Ulm (DE); MINICH, Waldemar, D-89134 Blaustein (DE)
(74) Vertreter: Andrae, Steffen
(86) Internationale Anmeldenummer: PCT/EP1999/000158
(87) Internationale Veröffentlichungsnummer: WO 1999/036552

(56) Entgegenhaltungen:
- WO-A-97/39131
- WO-A-98/20343
- S COSTAGLIOLA ET AL.: "Overexpression of the extracellular domain of the thyrotropin receptor in bacteria; production of the thyrotropin-binding inhibiting immunoglobulins" JOURNAL OF MOLECULAR ENDOCRINOLOGY, Bd. 13, Nr. 1, August 1994, Seiten 11-21, XP002103762
- W.B. MINICH ET AL.: "Expression of a biotinylated human thyrotropin receptor in HeLa cells using recombinant vaccinia virus and its application for the detection of Graves' autoantibodies" 70TH ANNUAL MEETING OF THE AMERICAN THYROID ASSOCIATION, Bd. 7, Nr. 1, 1997, Seite S-45 XP002103763 in der Anmeldung erwähnt
- W.B. MINICH ET AL.: "Expression of a functional tagged human thyrotropin receptor in HeLa cells using recombinantvaccinia virus" EXPERIMENTAL AND CLINICAL ENDOCRINOLOGY AND DIABETES, Bd. 105, Nr. 5, 1997, Seiten 282-290, XP002103764
- MORGENTHALER ET AL.: "In vitro synthesized TSH recptor as a tool for autoantibody detection" EXPERIMENTAL AND CLINICAL ENDOCRINOLOGY, Bd. 104, Nr. S 4, - 1996 Seiten 56-59,

## Beschreibung

Die vorliegende Erfindung betrifft Verfahren zur Herstellung von gereinigten und markierten TSH-Rezeptor-Präparaten, und die Verwendung derartiger Präparate in der Diagnostik.

Der Rezeptor für das Hormon TSH (thyroidstimulierendes Hormon), der TSH-Rezeptor (TSHR), spielt eine Schlüsselrolle für die Funktion und das Wachstum von Schilddrüsenzellen. Dieser Rezeptor ist ein Glied der Unterfamilie der G-Protein-gekoppelten Glykoproteinrezeptoren mit einer großen N-terminalen extrazellulären Domäne, die für die Ligandenbindung von essentieller Bedeutung ist und für die gezeigt wurde, daß sie an der Signalübermittlung beteiligt ist. Die Übermittlung des TSHR-Signals wird überwiegend durch die Aktivierung von Adenylatcyclase vermittelt, was zu einer Erhöhung des intrazellulären cAMP-Niveaus führt.

Ein Teil des großen Interesses an dem TSHR ist auf seine Rolle als primäres Autoantigen bei Morbus Basedow zurückzuführen, einer zu Schilddrüsenüberfunktion führenden Autoimmunerkrankung, die zu den häufigsten menschlichen Autoimmunerkrankungen überhaupt gehört. Der TSHR kommt in der Oberfläche von Schilddrüsenzellen nur in sehr geringen Mengen (in der Größenordnung von 1.000 - 10.000 Rezeptoren pro Zelle) vor.

Eine Aufklärung der chemischen Struktur des humanen TSH-Rezeptors (hTSHR), d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur). Seitdem wurden das hTSHR-Polypeptid und ausgewählte Teilpeptide davon in einer Vielzahl von Systemen exprimiert, wobei sich zeigte, daß ein funktionaler TSHR nur in Säugetierzellen hergestellt werden kann, und zwar sowohl transient als auch in Form einer stabilen Zellinie, sowie unter Verwendung von Adenovirus- und Vacciniavirus-Expressionssystemen. Aufgrund der Bedeutung des hTSHR als Autoantigen wurden in den letzten Jahren erhebliche Anstrengungen unternommen, das mittels Rekombinationstechniken erhaltene hTSHR-Protein (rhTSHR) zu reinigen.

Trotz zahlreicher Versuche wurden bisher keine überzeugenden Versuchsdaten bekannt, die zeigen, daß tatsächlich ein Verfahren entwickelt werden konnte, mit dem es möglich war, ein funktionales rhTSHR-Protein in solchen Mengen zu isolieren, wie sie im Hinblick auf das Binden pathologischer Autoantikörper gegen den TSHR (nachfolgend abgekürzt als TSHR-Auto-Ab) und deren klinischen Nachweis erforderlich wären. Eine erfolgreiche Reinigung wurde durch die relativ geringe Menge an TSHR bei der Expression in Säugetierzellen sowie durch die funktionelle Instabilität des Proteins behindert.

Ein wichtiger Faktor, der zur Schwierigkeit der Gewinnung eines in Assays verwendbaren gereinigten TSHR-Präparats beitrug, besteht darin, daß TSHR-Auto-Ab, ähnlich wie das Hormon TSH selbst, anscheinend vorwiegend hochkonformative Epitope erkennen, die durch Reinigungsversuche und auch Markierungsversuche leicht beeinträchtigt werden können.

Der Mangel an einem markierten, löslichen bzw. dispergierbaren hTSHR-Antigen verhinderte auch die Entwicklung von klinisch einsetzbaren direkten Immunpräzipitationsassays zum Nachweis von pathologischen hTSHR-Autoantikörpern.

Aus der Arbeit von N.G.Morgenthaler at al., Exp Clin Endocrinol Diabetes 104 (1996) Suppl 4, S.56-59 ist zwar ein Immunpräzipitationsassays für TSHR-Auto-Ab bekannt. Bei diesem Assay wird als Reagens zur Fällung der in einer Probe vorhandenen TSHR-Auto-Ab eine Präparation eines extrazellulären Teils eines rekombinanten, durch Einbau von ³⁵S-Methionin markierten TSH-Rezeptors verwendet. Ein solcher Assay hat den inhärenten Vorteil, daß weder eine Selektivität für stimulierende TSHR-Auto-Ab (TSI), noch für solche TSHR-Auto-Ab besteht, die mit bTSH kompetieren (TBII). Die Herstellung des ³⁵S-markierten Rezeptors durch in vitro-Translation ist jedoch außerordentlich aufwendig und teuer, und es gibt keine Meßgeräte, die für eine klinische Routinemessung der von ³⁵S emittierten Strahlung geeignet sind. Das Verfahren ist daher als Meßverfahren für die klinische Routinediagnostik nicht geeignet. Für einen klinisch einsetzbaren TSHR-Auto-Ab-Immunpräzipitationsassay würde ein funktionales hTSHR-Präparat benötigt, das mit einem der gängigen Label, insbesondere einem Radioisotop wie ¹²⁵I, markiert ist. Ein derartiges ¹²⁵I-rhTSHR-Präparat ist jedoch noch nicht verfügbar.

Der einzige gegenwärtig in der klinischen Praxis verwendbare Assay ist daher ein indirekt arbeitender Assay, bei dem die Inhibierung der Bindung von bTSH an einen mit Hilfe von Detergentien solubilisierten porcinen TSHR aus Schweine-Schilddrüsen zur Anwendung kommt. Ein direkter Immunpräzipitationsassay würde für die Diagnose des Morbus Basedow einen großen Vorteil darstellen, da er es erlauben könnte, alle Autoantikörper gegen den TSHR nachzuweisen, einschließlich derer, die an den TSHR binden, jedoch dabei die Wechselwirkung von TSH-Hormon bzw. dem Kompetitor bTSH mit dem TSHR nicht stören.

Auch für die Prüfung einer Anwendbarkeit des Autoantigens hTSHR in der Therapie standen bisher keine ausreichend reinen TSHR-Präparate in solchen Mengen zur Verfügung, daß damit reproduzierbare Tierexperimente oder klinische Untersuchungen möglich gewesen wären. Es ist nämlich aus Guimaraes VC et al., Endocrinology 1996, 137(6): 2199-2207 bekannt, daß man es im Tierversuch erreichen kann, durch orale Verabreichung des Schilddrüsen-Autoantigens Thyreoglobulin (TG) an Mäuse die Toleranz dieser Mäuse gegenüber dem gleichen Autoantigen erheblich zu erhöhen, wenn dieses anschließend injiziert wird. Auf dem "70th Annual Meeting of the American Thyroid Association", 15.-19.10.1997, (vgl. Abstract 206) wurde vorgetragen, daß auch beim Menschen durch orale TG-Verabreichung eine entsprechende Tolerisierung erreichbar zu sein scheint, die sich als spezifische Suppression der zellulären Immunantwort durch das vorher oral verabreichte Autoantigen äußert. Diese Ergebnisse lassen es hochinteressant erscheinen, auch andere Schilddrüsen-Autoantigene als TG auf eine im o.g. Sinne u.U. therapeutisch nützliche Anwendbarkeit zu testen. Dafür sind jedoch ausreichende Mengen des jeweiligen Autoantigens in hoher Reinheit erforderlich. Es war bisher kein Verfahren bekannt, das das Autoantigen hTSHR in der für derartige Versuche erforderlichen Reinheit/Konzentration verfügbar macht.

Es ist somit Aufgabe der vorliegenden Erfindung, ein Verfahren zur Herstellung eines gereinigten funktionalen hTSHR zu schaffen, bei dem dieser in flüssiger Phase frei von Fremdpeptidensequenzen und in markierter Form, insbesondere radiomarkierter Form, erhältlich ist.

Es ist ferner Aufgabe der vorliegenden Erfindung, neue diagnostische Verwendungen von hTSHR-Präparaten anzugeben, die durch die nach dem erfindungsgemäßen. Verfahren erhältlichen neuen gereinigten und markierten rhTSHR-Präparationen ermöglicht werden.

Diese Aufgaben werden durch ein Verfahren gemäß Anspruch 1 sowie die Verwendungen gemäß den Ansprüchen 7 und 8 gelöst.

Vorteilhafte, gegenwärtig bevorzugte Ausgestaltungen des erfindungsgemäßen Verfahrens gemäß Anspruch 1 werden in den Unteransprüchen 2 bis 6 wiedergegeben.

Gemäß der vorliegenden Erfindung wird ein Verfahren zur Reinigung und zum radioaktiven Markieren eines rekombinant erzeugten TSHR (rTHSR), insbesondere eines humanen TSHR (rhTSHR) geschaffen, bei dem von einem Fusions-TSHR, insbesondere einem biotinylierten Fusions-TSHR gemäß Patentanmeldung PCT/EP97/06127; "70th Annual Meeting of the American. Thyroid Association", 15. -19 -10.1997 (vgl. Abstract 89); bzw. W B Minich, J D Weymayer und U Loos, Thyroid, Vol. 8, S.3-7 (1998; im Druck) ausgegangen wird, wie er in HeLa-Zellen unter Verwendung eines rekombinanten Vacciniavirus exprimiert werden kann (vgl. auch W. B. Minich, M. Behr, U. Loos in: Exp. clin Endocrinol / Diabetes 105(1997) 282-290)). Der nach der vorliegenden Erfindung erstmals erhaltene ¹²⁵I-markierte funktionale rekombinante hTSHR (¹²⁵I-rhTSHR) wurde in einem direkten Immunopräzipitationsassay zum Nachweis und zur Bestimmung von pathologischen Autoantikörpern in unfraktionierten Seren von Patienten verwendet, die an Morbus Basedow litten.

Er ist auch für die Prüfung seiner therapeutischen Wirksamkeit bei einer oralen Tolerisierung von Patienten mit Morbus Basedow und endokriner Orbitopathie im o.g. Sinne geeignet und vorgesehen.

Eine solche Anwendung erscheint geeignet, den Autoimmunprozeß bei Morbus Basedow, insbesondere in Form seine Auswirkungen im Hinblick auf eine Ausbildung einer endokrinen Orbitopathie, günstig zu beeinflussen.

Im vorausgehenden und nachfolgenden Text dieser Anmeldung werden die verwendeten Reagenzien bzw. Analyten/Biomoleküle in der Regel mit verschiedenen Abkürzungen gekennzeichnet, die stets in den folgenden Bedeutungen zu verstehen sind, es sei denn, es ergibt sich für den Fachmann aus dem konkreten Zusammenhang etwas anderes. Die Verwendung der speziellen Angaben erfolgt dabei aus Gründen der exakten Beschreibung der durchgeführten Versuche und Messungen, bedeutet jedoch nicht, daß die beschriebenen Ergebnisse und Schlußfolgerungen nur für den beschriebenen Spezialfall gelten und nicht unter Heranziehung des relevanten Fachwissens verallgemeinert werden können.
- TSH =: Thyroidstimulierendes Hormon (Thyreotropin). Wird die Abkürzung TSH ohne weitere Zusätze verwendet, handelt es sich nicht um ein bestimmtes Produkt, sondern die Bindung bzw. Funktion des Hormons wird in allgemeiner Form diskutiert.
- bTSH =: Bovines (d.h. aus Rindern gewonnenes) TSH. Präparat, das in Assays zur Bestimmung von Autoantikörpern gegen den TSH-Rezeptor als Kompetitor eingesetzt wird, insbesondere in markierter Form (radioiodiert oder mit einem Chemilumineszenzlabel, insbesondere einem Acridiniumesterlabel, markiert).
- ¹²⁵I-bTSH =: Radioiodiertes, als Kompetitor eingesetztes bTSH. Im Zusammenhang mit der Beschreibung von Assays der Firma B.R.A.H.M.S Diagnostica GmbH steht ¹²⁵I-bTSH insbesondere für ein Produkt, wie es gemäß DE 42 37 430 C1 bzw. EP 0 668 775 B1 erhalten wird und Bestandteil des TRAK-Assay® der Fa. B.R.A.H.M.S Diagnostica GmbH ist.
- TSHR =: Der TSH-Rezeptor, ein in der Schilddrüsenmembran verankerter Glykoproteinrezeptor. Wird die Abkürzung TSHR ohne weitere Zusätze verwendet, handelt es sich nicht um ein bestimmtes Produkt, sondern die Funktion des Rezeptors bzw. seine Bindungsbeteiligung wird in allgemeiner Form diskutiert.
- porc.TSHR =: Aus Schilddrüsen von Schweinen (porcines) extraktiv gewonnenes krudes Rezeptorpräparat. Wird in den kompetitiven Rezeptor-Präzipitationsassays des Standes der Technik zur Be- stimmung von Autoantikörpern gegen den TSHR (herkömmlicher TRAK-Assay® der Fa. B.R.A.H.M.S Diagnostica GmbH) als spezifischer Binder eingesetzt.
- rhTSHR =: Gentechnisch erzeugtes (rekombinantes) Polypeptid, das die Aminosäuresequenz eines natürlich vorkommenden humanen TSHR wenigstens in einem solchen Ausmaße aufweist, daß es als "funktionaler humaner TSH-Rezeptor" bezeichnet werden kann, was bedeutet, daß es sich bezüglich der Bindung von Autoantikörpern gegen den TSHR bzw. von hTSH in signifikantem Ausmaß wie der natürlich vorkommende humane TSHR verhält. Wird die Abkürzung rhTSHR ohne weitere Zusätze verwendet, handelt es sich nicht um ein bestimmtes Produkt, d.h. rhTSHR kann für das rekombinante vollständige, mehr oder weniger glykosylierte Polypeptid, eine Teilsequenz einer ausreichenden Länge oder ein gentechnologisch erzeugtes Fusionsprodukt davon stehen (wie es z.B. in der Internationalen Patentanmeldung PCT/EP97/06121 beschrieben wird). Ohne weitere Erläuterungen liegt ein einfach als "rhTSHR" bezeichnetes Produkt als krude Membranpräparation vor, d.h. in der Form, wie sie durch konventionelle Solubilisierung von Membranen der zur Expression des rekombinanten Polypeptids verwendeten Zellen unter Verwendung von Detergenzien erhalten wird.
- rTSHR =: gentechnisch (rekombinant) erzeugter funktionaler TSHR mit einer Aminosäuresequenz eines TSHR beliebiger (humaner oder tierischer) biologischer Herkunft, der ansonsten genau wie der in den Beispielen konkret verwendete rhTSHR definiert, herstellbar und ggf. verwendbar ist.
- Fusions-TSHR =: rhTSHR mit angefügten Aminosäuresequenzen, die eine Polypeptidsequenz des natürlich vorkommenden funktionalen TSHR verlängern.
- rhTSHR-BIO =: biotinylierter Fusions-TSHR, dessen Herstellung und Verwendung in der älteren Anmeldung PCT/EP97/06121 beschrieben ist.
- rhTSHR-xa-BIO =: Um eine eingeschobene Angriffstelle für die Protease Xa (Faktor Xa) verlängerter rhTSHR-BIO.
- rhTSHR(rein) =: Durch Abdauen des an eine Festphase gebundenen rhTSHR-Xa-BIO mit der Protease Xa erhaltenes gereinigtes funktionales rhTSHR-Präparat.
- ¹²⁵I-rhTSHR =: Durch Abdauen des an eine Festphase gebundenen und in dieser Form radioiodierten rhTSHR-Xa-BIO mit der Protease Xa erhaltenes radioiodiertes rhTSHR-Präparat.
- Ab =: Antikörper
- TSHR-Auto-Ab =: In biologischen Proben, insbesondere humanem Serum oder Plasma, nachweisbare Autoantikörper gegen den TSH-Rezeptor. Ihr Nachweis ist mit bestimmten Schilddrüsen-Autoimmunerkrankungen korrelierbar. Der Nachweis stimulierender TSHR-Auto-Ab (in der Literatur auch ab- gekürzt TSI = thyroid stimulating immunoglobulins) ist insbesondere für die Diagnose des Morbus Basedow (englisch: Grave's disease) von Bedeutung. Ein kommerzieller Assay (Radiorezeptorassay) zur Bestimmung von TSHR-Auto-Ab ist der TRAK-Assay® der Fa. B.R.A.H.M.S Diagnostica GmbH.

Die Expression eines biotinylierten rhTSHR (rhTSHR-BIO) in HeLa-Zellen unter Verwendung eines rekombinanten Vacciniavirus ist in der älteren, nicht vorveröffentlichten Patentanmeldung PCT/EP 97/06121 bzw. der o.g. Veröffentlichung in Thyroid 1998 beschrieben. Auf den Inhalt der genannten beiden Schriften wird zur Ergänzung der vorliegenden Ausführungen ausdrücklich Bezug genommen. Unter Verwendung des biotinylierten Fusions-TSHR (rhTSHR-BIO) wurde ein abgewandelter biotinylierter TSH-Rezeptor hergestellt, der zwischen dem eigentlichen Polypeptid-Teil des TSHR und dem Rest, an den Biotin gebundenist, eine Schnittstelle für eine Protease, nämlich im vorliegenden Falle die Protease Xa, aufwies. Dieser abgewandelte biotinylierte rhTSHR (rhTSHR-Xa-BIO) ließ sich, wie für den Fusionsrezeptor rhTSHR-BIO beschrieben, immobilisieren.

Biotinylierte TSH-Fusionsrezeptoren werden in sehr wirksamer Weise an Streptavidinagarose gebunden, da Strepatividin eine außerordentlich hohe Affinität zu Biotin (K_{d} beträgt etwa 10⁻¹⁵ M) aufweist, die die stärkste bekannte, nicht-kovalente Bindung zwischen einem Protein und einem Liganden darstellt. In immobilisierter Form läßt sich ein biotinylierter Fusions-TSHR radioiodieren, z.B. mit ¹²⁵I, und aufgrund der Fixierung des radioiodierten Proteins an die Festphase läßt sich nicht-spezifische Restradioaktivität durch Waschen leicht ohne Proteinverlust entfernen. Es zeigte sich, daß der rhTSHR-Polypeptidteil des immobilisierten rhTSHR-Xa-BIO nach der Chloramin T/Na¹²⁵I-Methode radioiodierbar ist.

Andere bekannten Verfahren zur Radioiodierung, insbesondere die schonenderen derartigen verfahren, dürften ebenfalls anwendbar sein. Für eine nachfolgende Verwendung erforderliche Veränderungen des Reaktionsmediums, z.B. durch Austausch des Puffers, Zusatz rezeptorstabilisierender Lösungsbestandteile wie beispielsweise BSA o.ä., können auf einfache Weise auf der. Stufe der proteolytischen Abspaltung .(des "Abdauens") des radioiodierten ¹²⁵I-rhTSHR erfolgen.

Normalerweise ist es aufgrund der extrem festen Bindung biotinylierter Proteine an Avidin oder Streptavidin schwierig, einmal gebundene biotinylierte Proteine wieder von der Festphase abzulösen. Die erfindungsgemäß genutzte Abtrennung unter Ausnutzung einer speziell eingeführten Protease-Schnittstelle in den biotinylierten Fusionsrezeptor ermöglicht die Abtrennung des rhTSHR (rein) auf saubere, selektive Weise. In S. Costagliola at al., J Mol Endocrinol, 13 S.11-21 (1994) ist diese Schnittestelle Teil einer solubilisierten extrazellulären TSHR-Domäne. Die verwendete Protease, Faktor Xa, ist eine Serinprotease, die eine selektive Spaltung der Peptidbindung im Anschluß an die Tetrapeptidsequenz Ile-Glu-Gly-Arg.mit hoher Spezifität bewirkt (vgl. Nagai, K. und Thogersen, H. C. (1987), Methods Enzymol, 153, 461-469). Der durch Abspaltung aus einem festphasengebunden immobilisierten Rezeptor (rhTSHR-Xa-BIO) erhaltene ¹²⁵I-rhTSHR wurde zur Immunpräzipitationsanalyse von Seren von Morbus Basedow-Patienten verwendet. Es wurde festgestellt, daß ein direkter Immunpräzipitationsassay für pathologische Autoantikörper gegen den TSH-Rezeptor erhalten werden kann. Der Präzipitationsassay ist, da er nicht auf einer Konkurrenz der Autoantikörper mit dem Hormon TSH (in Form von bTSH) beruht, in der Lage, TSHR-Auto-Ab unabhängig von ihrer Fähigkeit, mit bTSH zu kompetieren, nachzuweisen.

Die Kombination zweier aufeinanderfolgender hochselektiver Reinigungsschritte in Form von. (a) der Immobilisierung des rhTSHR-Xa-BIO an Streptavidinagarose und (b) der selektiven Spaltung des gebundenen TSHR-Xa-BIO mit der Protease Xa führt zu Produkten, die das rhTSHR-Polypeptid in einer vorher nicht gekannten Reinheit frei von allen oder wenigstens den meisten Begleitsubstanzen enthalten (rhTSHR(rein)). Nach einer Radioiodierung des rhTSHR-Xa-BIO im immobilisierten Zustand führt die Spaltung zu einem Präparat, das den direkt radioiodierten rhTSHR (¹²⁵I-rhTSHR) enthält.

Nachfolgend wird die Erfindung anhand von Herstellungs- und Anwendungsbeispielen unter Bezugnahme auf eine Figur noch näher erläutert.

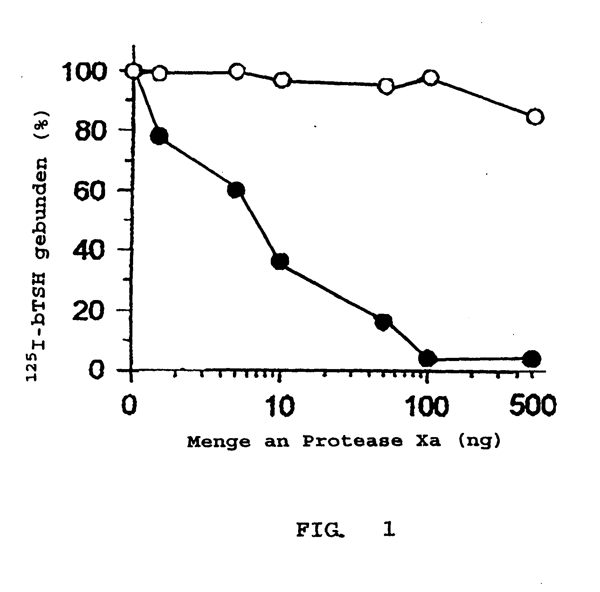

Die Figur (Figur 1) zeigt das verhalten des auf Streptavidin immobilisierten TSHR-BIO (-0-) bzw. TSHR-Xa-BIO (-•-) unter der Einwirkung der Protease Xa. Jeder der gezeigten Meßpunkte stellt den Mittelwert der jeweils eng beieinander liegenden Meßergebnisse einer Doppelbestimmung dar.

### Materialien und Methoden

### Materialien

¹²⁵I-bTSH (56µ-Ci/µg) sowie die für die Bestimmung verwendenten kommerziellen TRAK-Assay® -Kits wurden von der Firma B.R.A.H.M.S Diagnostica GmbH, Berlin zur Verfügung gestellt. Streptavidinagarose wurde als Handelsprodukt von der Firma Sigma bezogen. Die Protease Xa (Faktor Xa) wurde von der Fa. Böhringer Mannheim bezogen. Die DNA-Primer P1 (5'-GTCGGTTAC-CATGAAGGCCGCATGGAAGCGCCAGCAGCAG-3') und P2 (5'-CGCGGATCCT-TATTCGATAACAACAAGCGGTTC-3') wurden von der Firma Interactiva bezogen.

### Zellkultur

HeLa-Zellen wurden in Dulbecco's modifiziertem Eagle's Medium, das mit 10 % fetalem Rinderserum und 1mM Biotin ergänzt war, gezüchtet. Die Zellen wurden in einer 5%igen CO₂-Atmosphäre bei 37°C kultiviert.

### Ergänzung der hTSHR-Biotin-cDNA-Sequenzen durch eine Schnittstelle für Xa-Protease

Das Plasmid p7.5K131-TSHR-BIO (vgl. die internationale Patentanmeldung PCT/EP97/06121) enthält die Sequenz für die N-terminalen 725 Aminosäuren des humanen TSH-Rezeptors in Verknüpfung mit der 87 Aminosäuren langen C-terminalen Domäne der Biotin-Carboxyl-Carrier-Protein(BCCP)-Untereinheit der E. Coli-Acetyl-CoA-Carboxylase (vgl. die internationale Patentanmeldung PCT/EP97/06121, Versuchsreihe B.). Die BCCP-Sequenz wurde unter Anwendung der PCR-Technik unter Verwendung des Primers P1 (der eine BstEII-Schnittstelle und die Sequenz für eine Xa-Protease-Schnittstelle aufweist) und des Primers P2 (der ein Stopkodon sowie eine BamHI-Restriktionsstelle aufweist) vervielfältigt, wobei ein BstEIIBamHI-PCR-Fragment erhalten wurde. Das p7.5K131-TSHR-BIO-Plasmid wurde mit den Restriktasen BstEII und BamHI verdaut, das BstEII/BamHI-Fragment wurde entfernt und durch das BstEII/BamHI-PCR-Fragment ersetzt, wodurch das neue Vaccinia-Rekombinationsplasmid p7.5K131-TSHR-Xa-BIO erhalten wurde.

### Die Erzeugung von Vacciniavirus-Rekombinanten

Die TSHR-Xa-BIO-cDNA (aus dem p7.5K131-TSHR-Xa-BIO-Plasmid) wurde in das Genom des Wildtyp-Vacciniavirus-Stammes Copenhagen durch homologe Rekombination in das Thymidinkinasegen eingeschoben, wie detailliert in der älteren Anmeldung PCT/EP97/06121 beschrieben ist.

### Herstellung eines TSHR-Xa-BIO-Extrakts

Konfluente HeLa-Zellen, die in einer 75 cm²-Platte (ungefähr 20 x 10⁶ Zellen) gezüchtet wurden, wurden mit einer koloniebildenden Einheit (cfu) an rekombinantem Vacciniavirus pro Zelle infiziert und für 24 h bei 37°C inkubiert. Infizierte Zellen wurden mit einer phosphatgepufferten Salzlösung (PBS) gewaschen, geerntet und bei 1.500 U/min pelettiert. Die erhaltenen Zellen wurden in 0,3 ml eines Puffers A (20mM Hepes-KOH; pH 7,5; 50 mM NaCl; 1% Tween-20; 10 % Glycerol) unter Einfrieren und Auftauen lysiert. Die erhaltene Suspension wurde bei 30.000 g 1h zentrifugiert, der Überstand (etwa 8 mg/ml Gesamtprotein) wurde gesammelt und über Nacht gegen eine Puffer-A-Lösung dialysiert, um freies Biotin zu entfernen.

Ausgehend von dem Fusions-TSH-Rezeptor rhTSHR-BIO (vgl. PCT/EP 97/06121) wurde somit die für die N-terminalen 725 Aminosäuren des humanen TSHR, der in vollständiger Form 764 Aminosäuren umfaßt, kodierende DNA-Sequenz an eine DNA-Sequent angefügt, die für die 4 Aminosäuren umfassende Xa-Protease-Schnittstelle sowie die 87 Aminosäuren umfassende C-terminale Domäne der Biotin-Carboxyl-Carrier-Protein-(BCCP)- Untereinheit der E.-Coli- Acetyl-CoA-Carboxylase kodiert. Unter Verwendung des Vacciniavirus-Expressionssystems wurde das entsprechende Fusionsprotein (rhTSHR-Xa-BIO) auf gleiche Weise in HeLa-Zellen exprimiert, wie bereits früher für den biotinylierten Fusions-TSHR (rhTSHR-BIO) beschrieben wurde (vgl. PCT/EP 97/06121). Die mit in dem rekombinanten Virus infizierten HeLa-Zellen exprimierten etwa 120.000 Moleküle rhTSHR-Xa-BIO pro Zelle. Der exprimierte Rezeptor war vollständig funktional, d.h. er wechselwirkte mit TSH und erwies sich als aktiv im cAMP-Stimulationstest. Über den angefügten Biotinrest banden bei einem Verhältnis zu Streptavidinagarose von etwa 2,5 zu 1 (ml/ml) mehr als 90 % der Moleküle des rekombinanten Fusionsrezeptors rhTSHR-Xa-BIO an Streptavidinagarose.

Es zeigte sich, daß die Schnittstelle für die Protease Xa in dem rhTSHR-Xa-BIO-Protein einem Schneiden mit der Xa-Protease vollständig zugänglich war. Die Ergebnisse sind in Figur 1 gezeigt. Der an Streptavidinagarose gebundene biotinylierte rhTSHR-Xa-BIO wurde, nachdem er durch Waschen von kontaminierenden Proteinen befreit worden war, durch Messung seiner ¹²⁵I-bTSH-Bindungsaktivität mengenmäßig bestimmt. Wie in Figur 1 gezeigt ist, führt eine Behandlung mit der Xa-Protease zu einer vollständigen Entfernung von bTSH-bindenden Rezeptorproteinen von der Streptavidinagarose. Im Gegensatz dazu war der biotinylierte TSH-Rezeptor (TSHR-BIO) ohne die Schnittstelle für die Xa-Protease gegenüber einer Xa-Verdauung über einen weiten Bereich von Protease/Substrat-Verhältnissen unempfindlich.

### Reinigung und Markierung des proteolytisch gewonnenen ¹²⁵I-rhTSH-Rezeptors

Zur Gewinnung eines radiochemisch reinen ¹²⁵I-markierten rhTSHR ausgehend von mit dem rekombinanten Vacciniavirus infizierten HeLa-Zellen wurde eine dreistufige Arbeitsweise angewandt. Dabei wurden alle Operationen bei 4°C durchgeführt, es sei denn, es ist nachfolgend etwas anderes angegeben. Die Menge an rhTSHR-Protein wurde in jeder der Stufen aufgrund seiner ¹²⁵I-bTSH-Bindungsaktivität abgeschätzt.

### Stufe 1: Immobilisierung des biotinylierten TSHR-Xa-BIO an Streptavidinagarose

5 ml des TSHR-Xa-BIO-Extrakts aus HeLa-Zellen wurden über eine 0,5 ml Streptavidinagarose-Säule geleitet, die mit Puffer A äquilibriert war. Die Säule wurde mit 100 ml Puffer A sowie mit 5 ml eines 50 mM Natriumphosphatpuffers (pH 7,5) gewaschen, um kontaminierende Proteine zu entfernen. Unter den angegebenen Mengenverhältnissen von Extrakt zu Adsorptionsmittel wurden etwa 50 % des TSHR-Xa-BIO-Fusionsproteins an die Streptavidinagarose gebunden. Der Unterschied zwischen der ¹²⁵I-bTSH-Bindungskapazität des Ausgangsextrakts und der Durchflußfraktion betrug etwa 65.000 cpm.

### Stufe 2: ¹²⁵I-Markierung des an Streptavidinagarose gebundenen TSHR-Xa-BIO-Proteins

Die Streptavidinagarose mit dem daran gebundenen TSHR-Xa-BIO-Protein wurde in ein Eppendorf-Röhrchen überführt, und es wurden 1 mCi Na¹²⁵I und Chloramin T (bis zu einer Endkonzentration von 0,005 mg/ml) zugesetzt. Kontrollversuche hatten gezeigt, daß diese Menge an Chloramin T die TSH-Bindungsaktivität des immobilisierten rhTSHR-Xa-BIO-Proteins nicht beeinträchtigt. Die Mischung in einem Gesamtvolumen von 1 ml wurde unter Schütteln für, 1 Minute bei Raumtemperatur inkubiert. Die Reaktion wurde durch Zugabe von 2 µg Dithiothreit beendet. Das Harz wurde in eine Säule überführt und mit 50 mM Natriumphosphatpuffer (pH 7,5) und 100 ml Puffer A gewaschen, um nicht gebundenes radioaktives Material zu entfernen.

### Stufe 3: Abspaltung des ¹²⁵I-markierten rhTSH-Rezeptors von der Streptavidinagarose unter Verwendung von Xa-Protease

Die aus Stufe 2 erhaltene Streptavidinagarose mit den daran gebundenen Proteinen wurde mit 3 ml Puffer B (50 mM-Tris-HCl; pH 8;0; 100 mM NaCl; 1mM CaCl₂; 1% Triton X-100; 10 % Glycerol; 3 mg/ml Rinderserum-Albumin) gewaschen. Der ¹²⁵I-markierte rhTSHR wurde durch Inkubation unter anhaltendem Rühren der Säulenmatrix mit 125 ml Puffer B, der 3 µg Protease Xa enthielt, innerhalb von 12 h bei 4°C eluiert. Die Suspension wurde bei 14.000 U/min 10 min zentrifugiert, und der Überstand, der ¹²⁵I-rhTSHR enthielt, wurde gesammelt. Der erhaltene ¹²⁵I-rhTSHR wurde von niedermolekularem, ¹²⁵I-haltigem Material (wahrscheinlich Na¹²⁵I) dadurch befreit, daß man eine Spin-Filtration an Sephadex G-25 durchführte. Zu diesem Zweck wurden 1,25 ml der Proteinlösung auf die mit Puffer B äquilibrierte 6,5 ml Sephadex G-25-Säule aufgebracht. Die Säule wurde bei 2.000 U/min 5 min zentrifugiert, und die Durchflußfraktion (1,25 ml), die die ¹²⁵I-rhTSHR-Präparation darstellte, wurde in gleiche Teilmengen aufgeteilt und bei -70°C aufbewahrt.

Auf diese Weise wurde die Isolierung von 0,6 x 10⁶ cpm des ¹²⁵I-hTSHR-Proteins erreicht. Kontrollversuche (die unter identischen Bedingungen, jedoch ohne Na¹²⁵I durchgeführt wurden), zeigten, daß der isolierte Rezeptor aktiv war, indem er ungefähr 40.000 cpm ¹²⁵I-bTSH band. Die nicht-spezifische Wechselwirkung wurde in Gegenwart von 10⁻⁷ M TSH bestimmt und betrug < 10 % der Gesamtbindung. Aus der Messung der bTSH-Bindungsaktivität wurde geschlossen, daß die Ausbeute an rhTSHR etwa 30 % des Fusions-rhTSHR im ursprünglichen Zellextrakt sowie etwa 60 % des Fusions-rhTSHR, der auf Streptavidinagarose immobilisiert worden war, betrug. Wenn man (1) die Zellmenge, die für die 5 ml-Extraktherstellung eingesetzt worden war, (2) die Rezeptorzahl pro Zelle und (3) die Ausbeute an rhTSHR bei der Extraktherstellung berücksichtigt, läßt sich die Menge und spezifische Aktivität des isolierten ¹²⁵I-rhTSHR näherungsweise abschätzen. Sie sollte im Bereich von 1-2 µg und 0,1 bis 0,3 Ci/g (0,1 bis 0,3 x 10⁵ Ci/mol) liegen.

Die SDS-Elektrophorese von ¹²⁵I-rhTSHR zeigt ausschließlich eine Bande mit einer molaren Masse von etwa 90 kDA, was in guter Übereinstimmung mit der erwarteten molaren Masse des rhTSHR ist.

### Immunpräzipitationsanalyse

Für die Durchführung von Immunpräzipitationsversuchen wurden 10 µl ¹²⁵I-TSHR (etwa 4.000 cpm) mit 2,5 µl Patientenserum vermischt und 2 h bei Raumtemperatur inkubiert. Es wurde eine in Puffer A äquilibrierte Protein-A-Agarose zugesetzt (2,5 µl Bettvolumen der Aufschlämmung) und 1h bei Raumtemperatur inkubiert. Nach der Entfernung des Überstands wurde das Harz mit 0,3 ml Puffer A unter Zentrifugation gewaschen, und die Gammastrahlung im Pellet wurde gezählt. Die Ergebnisse wurden in Form der gebundenen Radioaktivität (cpm) erhalten.

### Bindungsassay für ¹²⁵I-bTSH sowie TSHR-Auto-Ab

Zur Gewinnung von Bezugswerten für die Immunpräzipitationsanalyse wurde ein kompetitiver Radiorezeptor-Präzipitatinsassay wie folgt durchgeführt: Das Binden von ¹²⁵I-bTSH sowie von damit kompetierenden TSHR-Auto-Ab aus Patientenseren an einen solubilisierten porcinen TSHR (oder an einen solubilisierten und immobilisierten rhTSHR-BIO) wurden unter Verwendung des TRAK-Assay® -Kits nach den Anweisungen der Hersteller durchgeführt. Der Assay wurde in einem Gesamtvolumen von 200 µl durchgeführt, das sich aus 50 µl porc.TSHR-Lösung, 50 µl des Testserums oder des Standard-Nullserums und 100 µl einer ¹²⁵I-bTSH-Lösung (ungefähr 25.000cpm) zusammensetzte. Die Inkubation wurde für 2 h bei Raumtemperatur durchgeführt, und die gebildeten ¹²⁵I-bTSH-porc.TSHR-Komplexe wurden durch Zugabe von 2 ml Polyethylenglykol (PEG) ausgefällt und durch Zentrifugieren bei 2000 g für 10 min abgetrennt, und die Gammastrahlung im erhaltenen Pellet wurde gezählt. Die Ergebnisse wurden ausgedrückt in Einheiten/Liter, unter Verwendung eines Radioaktivitäts-Konzentrationsprofils (Standardverdrängungskurve, erhalten mit Standardseren mit 0, 5, 15, 45, 135 und 405 Einheiten/Liter aus dem TRAK-Assay® -Kit). Die unspezifische Wechselwirkung wurde in Gegenwart von 10⁻⁷ M TSH bestimmt und betrug < 10 % der Gesamtbindung.

Im Falle des immobilisierten rhTSHR wurde der ¹²⁵I-bTSH-Bindungsassay wie oben beschrieben durchgeführt, außer daß 50 µl einer 20%igen Suspension von rhTSHR-BIO, gebunden an Streptavidinagarose, als Rezeptorpräparat eingesetzt wurden. Die Mischung wurde 2 h bei Raumtemperatur unter anhaltendem Rühren inkubiert. Nach der Entfernung des Überstands wurde das Harz mit 0,3 ml des Puffers A unter Zentrifugation gewaschen, und die Gammastrahlung im Pellet wurde gezählt. Die unspezifische Wechselwirkung wurde in Gegenwart von 10⁻⁷ M TSH bestimmt und betrug < 10 % der Gesamtbindung.

### Nachweis von Morbus Basedow-Autoantikörpern (TSHR-Auto-Ab) in Patientenseren unter Verwendung des ¹²⁵I-rhTSHR

Die wie oben erhaltene ¹²⁵I-rhTSHR-Präparation wurde zum Nachweis von pathologischen Autoantikörpern in Seren von Morbus Basedow-Patienten durch Immunpräzipitationsanalyse verwendet. Die Reaktionsmischung enthielt jeweils das Patienten-Testserum, ¹²⁵I-rhTSHR sowie Protein-A-Agarose. Am Ende des Inkubationszeitraums wurden die gebildeten Immunpräzipitate abzentrifugiert, und es wurde die Gammastrahlung im erhaltenen Pellet gemessen. Die gemessene Radioaktivität war proportional zur Menge an TSHR-Auto-Ab in den Patientenseren, wie sie in diesen Seren unabhängig nach den o.g. anderen Verfahren bestimmt worden war (TRAK-Assay® der Fa. B.R.A.H.M.S Diagnostica GmbH). Es wurde eine klare positive Korrelation zwischen dem Ergebnis des Immunpräzipitationsassays und den Ergebnissen des TRAK-Assays® für 52 Seren von Patienten mit Morbus Basedow unterschiedlicher Schwere sowie für 51 Seren gesunder Personen festgestellt. Im Immunpräzipitationsassay unter Verwendung des ¹²⁵I-rhTSHR wurden alle Seren von Morbus-Basedow-Patienten als positiv gemessen (Bindung im Bereich von 500 bis 1.000 cpm), wobei bis zu 25 % des zugesetzten ¹²⁵I-rhTSHR präzipitiert wurden. Die in der Reaktion verwendeten Autoantikörper-Konzentrationen entsprechen einem linearen Kurvenzug der Bindungskurve, wie sie mit dem TRAK-Assay® unter Verwendung stark positiver Morbus Basedow-Seren (220 U/l) erhalten wird. Kontrollseren binden ¹²⁵I-rhTSHR mit einer niedrigeren Wirksamkeit im Bereich von 100 bis 500 cpm.

Es ist dabei darauf hinzuweisen, daß in verschiedenen Kontrollproben große Unterschiede bezüglich der ¹²⁵I-rhTSHR-Bindungskapazität festgestellt wurden. Sollten derartige Unterschiede auf die Anwesenheit von Autoantiköpern zurückgeführt werden können, die zwar an rhTSHR binden, jedoch die Bindung von bTSH an rhTSHR in kompetitiven Assaydesigns nicht beeinträchtigen, so sollte die Bedeutung derartiger Antikörper für die Pathogenese des Morbus Basedow noch näher untersucht werden.

## Patentansprüche

1. Verfahren zur Herstellung eines direkt markierten gereinigten rekombinanten thyroids mulietendes Hormon-Rezeptorpraparats (rTSHR-Präparat), das sich bezüglich der Bindung von hTSH und Autoantikörpern gegen den humanen TSH-Rezeptor (hTSHR) wie der natürlich vorkommende humane TSHR verhält, bei dem man
- einen rekombinanten Fusions-TSH-Rezeptor (Fusions-TSHR) herstellt, der wenigstens aufweist (i) die Aminosäuresequenz des vollständigen humanen TSH-Rezeptors oder eines sich bezüglich der Bindung von hTSH und Autoantikörpern gegen den humanen TSH-Rezeptor (hTSHR) wie der natürlich vorkommende humane TSHR verhaltendes Fragments davon, (ii) einen diese Aminosäuresequenz verlängernden Peptidrest, der als solcher oder über einen an ihn gebundenen Biotinrest selektiv an eine geeignete Festphase gebunden werden kann, sowie (iii) zwischen der Aminosäuresequenz des TSH-Rezeptors und dem Peptidrest eine eingeschobene zusätzliche Aminosäuresequenz, die mit Hilfe einer geeigneten Protease selektiv von der Aminosäuresequenz des TSH-Rezeptors abspaltbar ist,
- den Fusionsrezeptor an eine geeignete Festphase bindet und durch waschen von kontaminierenden Bestandteilen befreit,
- den Fusionsrezeptor in festphasengebundender Form einer Umsetzung unterwirft, die zur Bindung eines Markierungsanteils direkt an die Aminosäuresequenz des TSH-Rezeptors führt,
- den an die Festphase gebundenen markierten Fusionsrezeptor mit einer Protease behandelt, die die Aminosäuresequenz des TSH-Rezeptors proteolytisch vom festphasengebundenen Rest des Fusionsrezeptors abspaltet, und
- das erhaltene direkt markierte gereinigte rTSHR-Präparat, das sich bezüglich der Bindung von hTSH und Autoantikörpern gegen den humanen TSH-Rezeptor (hTSHR) wie der natürlich vorkommende humane TSHR verhält, gewinnt.

2. Verfahren nach Anspruch 1, **dadurch gekennzeichnet, daß** die Aminosäuresequenz des TSH-Rezeptors die Aminosäuresequenz eines humanen TSH-Rezeptors ist.

3. Verfahren nach einem der Ansprüche 1 oder 2, **dadurch gekennzeichnet, daß** der Fusions-TSHR ein biotinylierter Fusions-TSHR (TSHR-Xa-BIO) ist, der einen biotinylierten Biotin-Carrierprotein-Peptidrest sowie eine eingeschobene Aminosäuresequenz aufweist, die durch die Protease Xa von der Aminosäuresequenz des funktionalen TSH-Rezeptors abspaltbar ist, und daß die Festphase eine Avidin- oder Streptavidin-Festphase ist.

4. Verfahren nach Anspruch 3, **dadurch gekennzeichnet, daß** die Festphase Streptavidin-Agarose ist.

5. Verfahren nach einem der Ansprüche 1 bis 4, **dadurch gekennzeichnet, daß** man zur Gewinnung eines radioiodierten rTSHR-Präparats den Fusions-TSHR in festphasengebundenem Zustand einer Umsetzung unterwirft, die zur Einführung von radioaktiven Iodieotopen in die Aminosäuresequenz des TSH-Rezeptors führt, und man nach der Umsetzung ungebundene radioaktive Bestandteile der Reaktionsmischung von den festphasengebundenen Bestandteilen abtrennt.

6. Verfahren nach Anspruch 5, **dadurch gekennzeichnet, daß** man den festphasengebundenen Fusions-TSHR auf an sich bekannte Weise mit Na ¹²⁵I/Chloramin T umsetzt.

7. In vitro Verwendung eines radioiodierten rTSHR-Präparats, wie es nach einem der Ansprüche 5 oder 6 erhältlich ist, als Reagens zur Detektion von TSH-Rezeptor-Autoantikörpern.

8. Verwendung eines radioiodierten funktionalen rTSHR-Präparats nach Anspruch 7 als Reagens zur Präzipitation von TSH-Rezeptor-Autoantikörpern in humanem Serum oder Plasma zum Zwecke ihres qualitativen Nachweises oder ihrer quantitativen Bestimmung.

## Claims

1. Method for the preparation of a directly labelled purified recombinant thyroid stimulating hormone receptor preparation (rTSHR preparation) which with respect to the binding of hTSH and of autoantibodies against the human TSH receptor (hTSHR) behaves in the same way as the naturally occurring human TSHR, in which
- a recombinant fusion TSH receptor (fusion TSHR) is prepared which at least comprises (i) the amino acid sequence of the complete human TSH receptor or of a fragment thereof which with respect to the binding of hTSH and autoantibodies against the human TSH receptor (hTSHR) behaves in the same way as the naturally occurring human TSHR, (ii) a peptide residue which extends this amino acid sequence and which, as such or via a biotin residue bound to it, can be selectively bound to a suitable solid phase, and (iii) between the amino acid sequence of the TSH receptor and the peptide residue, an inserted additional amino acid sequence which can be selectively cleaved from the amino acid sequence of the TSH receptor with the aid of a suitable protease,
- said fusion receptor is bound to a suitable solid phase and is freed from contaminating constituents by washing,
- the fusion receptor bound to the solid phase is subjected to a reaction which leads to the binding of a labelling portion directly to the amino acid sequence of the TSH receptor,
- the labelled fusion receptor bound to the solid phase is treated with a protease which cleaves the amino acid sequence of the functional TSH receptor proteolytically from the fusion receptor residue bound to the solid phase, and
- the obtained purified directly labelled rTSHR preparation which with respect to the binding of hTSH and autoantibodies against the human TSH receptor (hTSHR) behaves in the same way as the naturally occurring human TSHR is recovered.

2. Method according to Claim 1, **characterized in that** the amino acid sequence of the TSH receptor is the amino acid sequence of a human TSH receptor.

3. Method according to any of Claims 1 or 2, **characterized in that** the fusion TSHR is a biotinylated fusion TSHR (TSHR-Xa-BIO) which has a biotinylated biotin carrier protein peptide residue and an inserted amino acid sequence which can be cleaved by protease Xa from the amino acid sequence of the functional TSH receptor, and that the solid phase is an avidin or streptavidin solid phase.

4. Method according to Claim 3, **characterized in that** the solid phase is streptavidin agarose.

5. Method according to any of Claims 1 to 4, **characterized in that**, in order to obtain a radioiodinated rTSHR preparation, the fusion TSHR in the state bound to the solid phase is subjected to a reaction which leads to the introduction of radioactive iodine isotopes into the amino acid sequence of the TSH receptor, and unbound radioactive constituents of the reaction mixture are removed after the reaction from the constituents bound to the solid phase.

6. Method according to Claim 5, **characterized in that** the fusion TSHR bound to the solid phase is reacted with Na¹²⁵I/chloramine T in a manner known per se.

7. *In vitro* use of a radioiodinated rTSHR preparation obtainable according to any of Claims 5 or 6 as a reagent for the detection of TSH receptor autoantibodies.

8. Use of a radioiodinated functional rTSHR preparation according to Claim 7 as reagent for the precipitation of TSH receptor autoantibodies in human serum or plasma for their qualitative detection or their quantitative determination.

## Revendications

1. Procédé de fabrication d'une préparation purifiée et directement marquée de récepteurs TSH recombinés (préparation rTSHR), qui se comporte concernant la liaison du hTSH et des auto-anticorps contre le récepteur TSH humain (hTSHR) comme le TSHR humain naturel, dans laquelle
- on prépare une fusion recombinée du récepteur TSH (fusion-TSHR), qui présente au moins (i) la séquence des acides aminés du récepteur TSH humain complet ou d'un fragment de celle-ci, se comportant comme le TSHR humain naturel concernant la liaison du hTSH et des auto-anticorps contre le récepteur TSH humain (hTSHR), (ii) un reste peptidique allongeant cette séquence des acides aminés, qui peut être lié, comme tel ou par un reste biotine lié à celui-ci, sélectivement à une phase solide appropriée, ainsi que (iii) entre la séquence des acides aminés du récepteur TSH et le reste peptidique, une séquence des acides aminés supplémentaire intercalée, qui est clivable sélectivement de la séquence des acides aminés du récepteur TSH, à l'aide d'une protéase appropriée,
- on lie le récepteur fusionné à une phase solide appropriée et on la libère, par lavage, des constituants contaminants,
- on soumet le récepteur fusionné, sous forme liée à la phase solide, à une transformation, qui conduit à la liaison d'un partie de marquage, directement sur la séquence des acides aminés du récepteur TSH,
- on traite le récepteur fusionné marqué, lié à la phase solide, avec une protéase, qui clive la séquence des acides aminés du récepteur TSH, de manière protéolytique, du reste liant la phase solide de la fusion-récepteur, et
- on obtient la préparation rTSHR purifiée, directement marquée, obtenue, qui se comporte concernant la liaison du hTSH et des auto-anticorps contre le récepteur TSH humain (hTSHR) comme le TSHR humain naturel.

2. Procédé selon la revendication 1, **caractérisé en ce que** la séquence des acides aminés du récepteur TSH est la séquence des acides aminés du récepteur TSH humain.

3. Procédé selon la revendication 1 ou 2, **caractérisé en ce que** la fusion-TSHR est une fusion-TSHR biotinylée (TSHR-Xa-BIO), qui présente un biotine-protéine support-reste peptidique biotinylé ainsi qu'une séquence des acides aminé intercalée, qui est clivable par la protéase Xa de la séquence des acides aminés du récepteur TSH fonctionnel et **en ce que** la phase solide est une phase solide avec avidine ou streptavidine.

4. Procédé selon la revendication 3, **caractérisé en ce que** la phase solide est streptavidine-agarose.

5. Procédé selon l'une des revendications 1 à 4, **caractérisé en ce que** pour l'obtention d'une préparation de rTSHR radiomarquée, on soumet la fusion-TSHR en l'état lié à la phase solide, à une transformation qui conduit à l'introduction d'isotopes radioactifs d'iode dans la séquence des acides aminés du récepteur TSH et **en ce que** l'on sépare après la transformation, les constituants radioactifs non liés du mélange réactionnel, depuis les constituants liés à la phase solide.

6. Procédé selon la revendication 5, **caractérisé en ce que** l'on transforme la fusion-TSHR liée à la phase solide, de manière connue, avec le Na¹²⁵I/chloramine T.

7. Utilisation d'une préparation de rTSHR radio-iodé, telle qu'elle est obtenue selon une des revendications 5 ou 6, comme réactif pour la détection d'auto-anticorps du récepteur TSH.

8. Utilisation d'une préparation de rTSHR fonctionnel radio-iodé selon la revendication 7, comme réactif pour la précipitation d'auto-anticorps du récepteur TSH dans le sérum ou le plasma humain pour leur détection qualitative ou leur estimation quantitative.
